# EUROPEAN PATENT APPLICATION

(11) **EP 4 527 402 A1**
(43) Date of publication of application: **26.03.2025**
(21) Application number: 24201364.7
(22) Date of filing: 19.09.2024
(51) Int. Cl.: A61K 36/53, A61K 36/71, A61P 11/00, A61K 31/192

(54) **COMPOSITIONS AND METHODS FOR IMPROVING LUNG FUNCTION**

(30) Priority: 19.09.2023 IN 202341062803
(71) Applicant: Majeed, Muhammed, Bangalore 560058 (IN); Nagabhushanam, Kalyanam, East Windsor, NJ 08520 (US); Mundkur, Lakshmi, Bangalore Karnataka 560058 (IN); Syed, Musthafa K, Bangalore Karnataka 560058 (IN); Veeranna, Huruli, Bangalore Karnataka 560058 (IN)
(72) Inventor: Majeed, Muhammed, 560058 Bangalore (IN); Nagabhushanam, Kalyanam, East Windsor, NJ New Jersey 08520 (US); Mundkur, Lakshmi, 560058 Bangalore (IN); Musthafa K, Syed, 560058 Bangalore (IN); Veeranna, Huruli, 560058 Bangalore (IN); Bani, Sarang, 560058 Bangalore (IN); Pandey, Anjali, 560058 Bangalore (IN)
(74) Representative: Grünecker Patent- und Rechtsanwälte PartG mbB

(57) **Abstract**

The present invention discloses a process of isolating Rosmarnic acid and a composition comprising Rosmarinic acid and *Nigella sativa* extract standardised to contain not less than 5% w/w thymoquinone. More specifically, the invention discloses the use of a composition comprising Rosmarinic acid and *Nigella sativa* extract standardised to contain not less than 5% w/w thymoquinone in improving lung function and delaying lung senescence in aging mammals.

## Description

### FIELD OF INVENTION

The present invention in general relates to compositions for the management of lung ageing and improving lung function. More specifically, the invention relates to compositions comprising Rosmarinic acid and *Nigella sativa* extract for improving lung function in compromised subjects and to prevent lung senescence. The invention further relates to a process of isolating Rosmarinic acid (RA) from plant part of Lamiaceae family and a process of preparing the plant part of Lamiaceae family suitable for the isolation of Rosmarinic acid.

### BACKGROUND OF THE INVENTION

Ageing is one of primary factors for the development of many diseases and disorders. The lung is one of the few internal organs continuously exposed to the external stimuli, underscoring the importance of environmental factors crucial to lung health. These factors include active or passive cigarette smoke, indoor occupational or household exposures (such as fumes from cooking or heating and toxins), pollen, and bacterial or viral pathogens, among others. Specialized immune cells in the lung, such as alveolar macrophages, neutrophils, and lung-resident dendritic cells, along with structural cells like bronchial and alveolar epithelial cells, typically neutralize or counteract these environmental stressors. However, prolonged or excessive exposure to these challenges, combined with epigenetic changes and genetic predisposition, can disrupt the balance of physiological repair and renewal, leading to the development of chronic lung diseases *(*Meiners et al., Hallmarks of the ageing lung, European Respiratory Journal 2015 45: 807-827; DOI: 10.1183/09031936.00186914*)* like Chronic obstructive lung disease, asthma, bronchitis, bronchiectasis, emphysema, Interstitial lung disease etc. The lung function in these individuals is generally compromised and impaired. Ageing also causes impaired lung function in healthy individuals, marked by structural changes that impair gas exchange and immunologic changes that predispose to infections (Schneider et al., The aging lung: Physiology, disease, and immunity, Cell; 2021; 184(8):1990-2019*).*

The effect of ageing on the lungs is multi-fold. Age-related changes in the lungs include (Rebecca Dezube, Effects of Aging on the Respiratory System, HEALTHY LIVING, Merck Manual, January 2023*):*
**a)** Decreased peak airflow (how quickly someone can exhale) and exchange of carbon dioxide and oxygen
**b)** Decreased lung function such as vital capacity (the maximum amount of air that can be breathed out following a maximum inhalation)
**c)** Weakened respiratory muscles
**d)** Reduced effectiveness of lung defense mechanisms

Further, ageing also stiffens the rib cage, reduce the elasticity of bronchial tubes and air sacs, desensitize the coughing nerves, toxins accumulates and resistance declines and increases risk of inflammation (Colleen M. Story, How Growing Older Impacts Lung Health, Helathline, Medically reviewed by Elaine K. Luo, MD. September 18, 2017*).* Further, age-related changes in the lungs are compounded by the effects of heart and lung diseases. Hence it is imperative to maintain proper lung function in compromised individuals and older adults.

There are many senolytic drugs that are being tested to counter the effects of lung ageing and improving lung function. Benidipine is reported to decrease senescent lung cells and ameliorates lung emphysema in a mouse model (Palazzo et al., Benidipine calcium channel blocker promotes the death of cigarette smoke-induced senescent cells and improves lung emphysema, Aging (Albany NY), 2023;15(23):13581-13592*).* Dasatinib and Quercetin showed promise in alleviating physical dysfunction in Idiopathic pulmonary fibrosis (IPF), however, a larger randomized controlled trials for senescence-related diseases for the ingredients were warranted (Justice et al., Senolytics in idiopathic pulmonary fibrosis: Results from a first-in-human, open-label, pilot study, EBioMedicine. 2019 Feb; 40: 554-563.). There exists an unmet industrial need to find effective natural ingredients or a combination thereof to improve lung function in compromised individuals and to promote healthy ageing of the lungs. The present invention addresses the above need by disclosing a composition comprising Rosmarinic acid, individually and in combination with *Nigella sativa* extract to reduce the signs and symptoms of lung ageing.

Numerous reports exist of the isolation and purification of Rosmarinic acid form different plant sources. However, due to its unstable nature, it degrades rapidly from the plant parts during the process of drying and storage, thereby reducing the total yields of Rosmarinic acid during extraction. The present invention solves the above industrial problem by disclosing a novel method for stabilizing the content of Rosmarinic acid in the plant material and preparing the plant material suitable for the isolation and purification of Rosmarinic acid from the plants of Lamiacea family.

It is the principal object of the invention to disclose a composition comprising Rosmarinic acid and *Nigella sativa* extract standardised to contain not less than 5% w/w thymoquinone.

It is another principal object of the invention to disclose a process for stabilizing the content of Rosmarinic acid in the plant material and preparing the plant material suitable for the isolation and purification of Rosmarinic acid from the plants of Lamiacea family.

It is another object of the invention to disclose the use of a composition comprising Rosmarinic acid and *Nigella sativa* extract standardised to contain not less than 5% w/w thymoquinone to improve lung function in ageing mammals.

It is another object of the invention to disclose the use of a composition comprising Rosmarinic acid and *Nigella sativa* extract standardised to contain not less than 5% w/w thymoquinone to prevent signs and symptoms of lung senescence.

The present invention solves the above-mentioned objects and provides related advantages.

### SUMMARY OF THE INVENTION

In the most preferred embodiment of the invention discloses a process of preparing plant part of Lamiaceae family, suitable for isolation of Rosmarinic acid comprising steps of:
a) Charging the plant part from Lamiaceae family into Masher machine;
b) Preparing acid solution (1 M - 5 M) and slowly charge the solution to Masher machine along with the plant parts to make uniform mixture;
c) Collecting the paste and leave the paste to dry for 3-5 hrs;
d) Charging the dried paste from step c to Masher machine;
e) Preparing base solution 0.05 M - 2.5 M and slowly charge the solution to Masher machine along with plant paste from step d to make uniform mixture;
f) Collecting the paste from step e and spread them on open yard for sun drying;
g) Collecting the dried material (paste coarse powder) from step f for isolation of Rosmarinic acid.

In another preferred embodiment of the invention discloses a process of isolating RA from the plant parts of Lamiaceae family preserved and stabilized as in previous embodiment
a) Charging 3 volumes of solvent to the reactor containing coarse powder of leaves and stirred at 60-65°C for three hours;
b) Filtering the mass from step a through Nutsche filter;
c) Repeating steps a and b for two more times
d) Collecting all filtrates and concentrated into a thick paste and stripping off all the solvent;
e) Dissolving the concentrated extract from step d with 12 volumes of demineralized water (DM) and stirring for 2-4 hrs at 50-65°C, then cool it to room temperature;
f) Adjusting pH to 2.8 - 3.2 with 10% Sodium bicarbonate solution / 50% Hydrochloric acid (HCl) and stirring for 15 mins, reconfirming the pH and filtering through hyflo bed;
g) Extracting the aqueous layer with one third volume of solvent, thrice;
h) Concentrating the solvent portion completely and noting down the quantity;
i) Dissolving the material obtained from Step h with 10 volumes of demineralised water and adjusting pH of the aqueous portion to 6.8 to 7.2 with 10% Sodium bicarbonate solution and filtering the mass through hyflo bed;
j) Partitioning the aqueous portion with one-third volume of solvent, thrice and separating the organic layer for solvent recovery;
k) Adjusting the pH to 2.8 to 3.2 with 50% HCl of the aqueous portion obtained from step j;
l) Passing the aqueous portion through Hyflo bed;
m) Partitioning the aqueous portion obtained from step l with one-third volume of solvent, thrice;
n) Discarding the aqueous layer after confirming the yield, concentrate solvent to pourable mass, and dry in Vacuum tray dryer (VTD) at 60-65 ° C to get powder;
o) Extracting the above material with 12 volumes of 20% salt-water solution, and repeated 3 times at 80-85 °C, each extraction 2 hrs.;
p) Combining all the aqueous extract and pass through Hyflo bed;
q) Extracting the clear filtrate with one-third volume of solvent (× 3 times);
r) Combining all solvent portion obtained from step q and wash with cold water;
s) Distilling off the solvent completely and dry at 60-65°C to get powder;
t) Charging 12 volumes of demineralised water, stirring at room temperature until complete solubilization of the extract;
u) Reducing the volume to six and storing at 4-5°C for 48 hrs;
v) Filtering the crystals and washing with cold water. Drying the product for 5-6hrs at room temperature and final drying at 65°C in VTD (Vacuum tray drier);
w) Isolating Rosmarinic acid with an yield of at least 0.5 kg as a Creamish white crystalline powder at not less than 90% w/w.

In another preferred embodiment, the invention discloses a composition comprising Rosmarinic acid and *Nigella sativa* extract standardised to contain not less than 5% w/w thymoquinone.

In another most preferred embodiment, the invention discloses a method for improving lung function in a subject comprising a) identifying a subject with decreased lung function, b) administering an effective dose of a composition comprising Rosmarinic acid, to bring about improvement in lung function.

In another most preferred embodiment, the invention discloses a method for delaying lung senescence in an ageing subject comprising a) identifying an aged subject showing signs of lung ageing, b) administering an effective dose of a composition comprising Rosmarinic acid, to bring about reduction in signs of lung ageing.

In another most preferred embodiment, the invention discloses a composition comprising Rosmarinic acid for use or use of a composition comprising Rosmarinic acid for improving lung function in a subject.

In another most preferred embodiment, the invention discloses a composition comprising Rosmarinic acid for use or use of a composition comprising Rosmarinic acid for delaying lung senescence in an ageing subject.

### BRIEF DESCRIPTION OF DRAWINGS

Fig. 1 is a graphical representation showing the increase in tidal volume in aging mice after administering either Rosmarinic acid or *Nigella sativa* extract (Nigellin^{®}) individually or as a combination. Rapamycin is used as a positive control (** p<0.05, **p<0.01).
Fig. 2 is a graphical representation showing the increase in minute ventilation in aging mice after administering either Rosmarinic acid or *Nigella sativa* extract (Nigellin^{®}) individually or as a combination. Rapamycin is used as a positive control (* p<0.05).
Fig. 3 is a graphical representation showing the increase in Transdiaphragmatic pressure (Pdimax) in aging mice after administering either Rosmarinic acid or *Nigella sativa* extract (Nigellin^{®}) individually or as a combination. Rapamycin is used as a positive control (* p<0.05, **p<0.01).
Fig. 4 is a graphical representation showing the modulating in respiratory rate in aging mice after administering either Rosmarinic acid or *Nigella sativa* extract (Nigellin^{®}) individually or as a combination. Rapamycin is used as a positive control (**p<0.01).
Fig. 5 is a graphical representation showing the increased expression of laminin in aging mice after administering either Rosmarinic acid or *Nigella sativa* extract (Nigellin^{®}) individually or as a combination. Rapamycin is used as a positive control (* p<0.05, **p<0.01).
Fig. 6 is a graphical representation showing the increased expression of SIRT-1 in aging mice after administering either Rosmarinic acid or *Nigella sativa* extract (Nigellin^{®}) individually or as a combination. Rapamycin is used as a positive control (* p<0.05, **p<0.01).
Fig. 7 is a graphical representation showing the increased expression of AMPK in aging mice after administering either Rosmarinic acid or *Nigella sativa* extract (Nigellin^{®}) individually or as a combination. Rapamycin is used as a positive control (**p<0.01).
Fig. 8 is a graphical representation showing the decrease in expression of mTOR in aging mice after administering either Rosmarinic acid or *Nigella sativa* extract (Nigellin^{®}) individually or as a combination. Rapamycin is used as a positive control (* p<0.05, **p<0.01.
Fig. 9 is a graphical representation showing the increased expression of IgA in aging mice after administering either Rosmarinic acid or *Nigella sativa* extract (Nigellin^{®}) individually or as a combination. Rapamycin is used as a positive control (* p<0.05, **p<0.01).

### DETAILED DESCRIPTION OF THE PREFERRED EMBODIMENTS

### Selected Definitions

All the terms used in this application carry ordinary meaning as known in the prior art unless otherwise specified. Few other specific definitions used in this invention are explained below, which applies throughout this specification. Claims provide broader definition unless and otherwise specified.

Furthermore, the terms "approximately," "approximate," "about," and similar terms generally refer to ranges that include the identified value within a margin of 20%, 10%, or preferably 5%, and any values there between.

As used herein, treatment or management of a condition refers to effectively ameliorating conditions disclosed in the invention.

As used herein, an effective dose refers to the concentration of an active ingredient which brings about positive or modulatory effects of a condition in a subject covered under this invention.

As used herein, % w/w refers to the purity of the ingredient.

As used herein, Pdimax refers to Transdiaphragmatic pressure, which represents the pressure across the diaphragm.

As used herein, Sirt-1 refers to Silencing information regulator 2 related enzyme 1.

As used herein, mTOR refers to mammalian target of rapamycin

As used herein, ageing broadly refers to biological or physical ageing

As used herein, Nigellin^{®} is a branded ingredient with standardized extract from Nigella Sativa.

As used herein, Rosinin^{™} is a branded ingredient for Rosmarinic acid

As used herein, Rosmagellin^{™} is a branded product containing the combination of Rosmarinic acid (RA) and *Nigella sativa.*

In a most preferred embodiment, the invention discloses a composition comprising Rosmarinic acid and *Nigella sativa* extract standardised to contain not less than 5% w/w thymoquinone .

In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables.

In the most preferred embodiment of the invention discloses a process of preparing plant part of Lamiaceae family, suitable for isolation of Rosmarinic acid comprising steps of:
a) Charging the plant part from Lamiaceae family into Masher machine;
b) Preparing acid solution (1 M - 5 M) and slowly charge the solution to Masher machine along with the plant parts to make uniform mixture;
c) Collecting the paste and leave the paste to dry for 3-5 hrs;
d) Charging the dried paste from step c to Masher machine;
e) Preparing base solution (0.05 M - 2.5 M) and slowly charge the solution to Masher machine along with plant paste from step d to make uniform mixture;
f) Collecting the paste from step e and spread them on open yard for sun drying;
g) Collecting the dried material (paste coarse powder) from step f for isolation of Rosmarinic acid.

In related embodiment of the invention wherein isolation of Rosmarinic acid from Lamiaceae family is selected from the family consisting essentially of Coleus leaves, Clinopodium chinense var. parviflorum, Clinopodium tomentosum (Kunth) Govaerts, Clinopodium urticifolium, Coleus aromaticus Benth, Coleus forskohlii (Willd) Briq., Coleus parvifolius Benth, Dracocephalum fruticulosum Steph. Ex Willd, Dracocephalum heterophyllum, Dracocephalum nutans L., Dracocephalum palmatum Stephan, Dracocephalum tanguticum Maxim., Elsholtiza bodinieri Vaniot, Elsholtzia rugulosa Hemsl., Elsholtzia splendens Nakai, Hypenia salzmannii (Benth.) Harley, Hyptis atrorubens Poit., Hyptis capitata Jacq., Hyptis pectinata (L.) Poit, Hyptis suaveolens (L.) Poit, Hyptis verticillata Jacq., Hyssopus cuspidatus, Isodon flexicaulis C. Y. Wu et H. W. Li, Isodon lophanthoides var. graciliflorus, Isodon oresbius (W. W. Smith) Kudo, Isodon rubescens (Hemsl.) Hara, Isodon rugosus (Wall. Ex Benth.) Codd, Isodon sculponeata (Vaniot) Hara, Keiskea japonica Miq., Lallemantia iberica (Bieb.) Fisch & C.A. Mey, Lavandula angustifolia Mill. Lepechinia graveolens (Reg.) Epling., Lepechinia meyenii (Walp.) Epling, Lepechinia speciosa (St. Hill) Epling, Lycopus europaeus L., Lycopus lucidus Turcz., Marrubium vulgare L., Meehania urticifolia (Miq.) Makino, Melissa officinalis L., Mentha dumetorum, Mentha haplocalyx Briq, Mentha piperita L., Mentha spicata L., Mesona chinensis Benth, Micromeria myrtifolia Boiss. & Hohen. Nepeta asterotricha Rech. F., Nepeta cadmea Boiss. Nepeta curviflora Boiss., Ocimum campechianum Mill, Ocimum sanctum Linn., Ocimum tenuiflorum, Origanum dictamnus L., Origanum glandulosum Desf, Origanum majorana L., Origanum minutiflorum, Origanum rotundifolium Boiss., Origanum vulgare L. ssp. Hirtum, Perilla frutescens (L.) Britton var. acuta Kudo, Perilla frutescens var. acuta, Perovskia atriplicifolia Benth, Plectranthus forsteri 'Marginatus', Plectranthus hadiensis var. tomentosus, Plectranthus madagascariensis (Pers.) Benth, Plectranthus scutellarioides (L.) R. Br., Prunella vulgaris L., Prunella vulgaris var. lilacina, Rosmarinus officinalis L.. Salvia absconditiflora Greuter & Burdet, Salvia castanea Diels f. tomentosa Stib., Salvia cavaleriei Levi., Salvia cerino-pruinosa Rech. F. var cerino-pruinosa, Salvia chinensis Benth, Salvia deserta Schang, Salvia flava Forrest, Salvia grandifoliaW. W. Smith, Salvia kiaometiensis Lévl., Salvia limbata C.A. Meyer, Salvia miltiorrhiza Bunge, Salvia officinalis, Salvia palaestina Bentham, Salvia plebeia R. Br., Salvia przewalskii Maxim, Salvia sonchifolia C.Y. Wu, Salvia splendens Sellow ex Roem & Schult, Salvia trichoclada Bentham, Salvia viridis L. cvar. Blue Jeans, Salvia yunaansis, Satureja biflora, Schizonepeta tenuifolia Briquet, Sideritis albiflora, Sideritis leptoclada, Solenostemon monostachys Briq, Thymus alternans Klokov, Thymus atlanticus (Ball) Roussine, Thymus praecox subsp grossheimii (Ronniger) Jalas, Thymus praecox subsp grossheimii (Ronniger ) Jalas var. grossheimii, Thymus quinquecostatus var. japonica, Thymus serpyllum, Thymus sibthorpii Bentham, Thymus sipyleus subsp. Sipyleus var. sipyleus, Thymus vulgaris L., or Ziziphora clinopodioides Lam. In related embodiment of the invention, plant part is selected from the group consisting of leaves, root, stem, or fruit. In a related embodiment, the acid is selected from the group consisting of, but not limited to, hydrochloric acid, sulphuric acid, formic acid, acetic acid. In another related embodiment, the base is selected from the group consisting of sodium hydroxide, potassium hydroxide, sodium carbonate, sodium bicarbonate, potassium carbonate, potassium bicarbonate, calcium hydroxide.

In another preferred embodiment of the invention discloses a process of isolating RA from the plant parts of Lamiaceae family preserved and stabilized as in previous embodiment
a) Charging 3 volumes of solvent to the reactor containing coarse powder of leaves and stirred at 60-65°C for three hours;
b) Filtering the mass from step a through Nutsche filter;
c) Repeating steps a and b for two more times
d) Collecting all filtrates and concentrated into a thick paste and stripping off all the solvent;
e) Dissolving the concentrated extract from step d with 12 volumes of demineralized water (DM) and stirring for 2-4 hrs at 50-65°C, then cool it to room temperature;
f) Adjusting pH to 2.8 - 3.2 with 10% Sodium bicarbonate solution / 50% Hydrochloric acid (HCl) and stirring for 15 mins, reconfirming the pH and filtering through hyflo bed;
g) Extracting the aqueous layer with one third volume of solvent, thrice;
h) Concentrating the solvent portion completely and noting down the quantity;
i) Dissolving the material obtained from Step h with 10 volumes of demineralised water and adjusting pH of the aqueous portion to 6.8 to 7.2 with 10% Sodium bicarbonate solution and filtering the mass through hyflo bed;
j) Partitioning the aqueous portion with one-third volume of solvent, thrice and separating the organic layer for solvent recovery;
k) Adjusting the pH to 2.8 to 3.2 with 50% HCl of the aqueous portion obtained from step j;
l) Passing the aqueous portion through Hyflo bed;
m) Partitioning the aqueous portion obtained from step l with one-third volume of solvent, thrice;
n) Discarding the aqueous layer after confirming the yield, concentrate solvent to pourable mass, and dry in Vacuum tray dryer (VTD) at 60-65 ° C to get powder;
o) Extracting the above material with 12 volumes of 20% salt-water solution, and repeated 3 times at 80-85 °C, each extraction 2 hrs.;
p) Combining all the aqueous extract and pass through Hyflo bed;
q) Extracting the clear filtrate with one-third volume of solvent (× 3 times);
r) Combining all solvent portion obtained from step q and wash with cold water;
s) Distilling off the solvent completely and dry at 60-65°C to get powder;
t) Charging 12 volumes of demineralised water, stirring at room temperature until complete solubilization of the extract;
u) Reducing the volume to six and storing at 4-5°C for 48 hrs;
x) Filtering the crystals and washing with cold water. Drying the product for 5-6hrs at room temperature and final drying at 65°C in VTD (Vacuum tray drier);
v) Isolating Rosmarinic acid with a yield of at least 0.5 kg as a Creamish white crystalline powder at not less than 90% w/w.

In a preferred embodiment, the solvent of step a) is selected from the group consisting of methanol, ethanol, Isopropyl alcohol, or acetone. In a preferred embodiment, the solvent of step a) is preferably ethanol. In another preferred embodiment, the solvent of steps g, j, m, n, r and s is selected form the group consisting of methanol, ethanol, ethyl acetate, choloroform, diethyl ether, acetone, hexane, dichloromethane, butanol, propanol. In a preferred embodiment, the solvent of g, j, m, n, r and s is preferably ethyl acetate.

In another most preferred embodiment, the invention discloses a method for improving lung function in a subject comprising a) identifying a subject with decreased lung function, b) administering an effective dose of a composition comprising Rosmarinic acid, to bring about improvement in lung function. In a related embodiment, the composition further comprises *Nigella sativa* extract standardised to contain not less than 5% w/w thymoquinone. In a related embodiment, lung function is decreased in conditions selected from the group consisting of, but not limited to, restrictive, obstructive or mixed lung disease or ageing. In another related embodiment, restrictive lung diseases include Idiopathic pulmonary fibrosis (IPF), Non-specific interstitial pneumonia (NSIP), Cryptogenic organizing pneumonia (COP), Sarcoidosis, Acute interstitial pneumonia (AIP), decrease in lung function due to inorganic dust exposure such as silicosis, asbestosis, talc, pneumoconiosis, berylliosis, hard metal fibrosis, coal worker's pneumoconiosis, chemical worker's lung, Organic dust exposure such as farmer's lung, bird fancier's lung, bagassosis, and mushroom worker's lung, humidifier lung, hot tub pneumonitis, Hypersensitivity pneumonitis, Systemic sclerosis, Pulmonary vasculitis, Pulmonary Langerhans cell histiocytosis, conditions due to medications such as nitrofurantoin, amiodarone, gold, phenytoin, thiazides, hydralazine, bleomycin, carmustine, cyclophosphamide, methotrexate and conditions due to radiation therapy. In another related embodiment, obstructive lung disease include Chronic obstructive pulmonary disease (COPD), which includes emphysema and chronic bronchitis, Asthma, Bronchiectasis and Cystic fibrosis. In a related embodiment, the signs of decreased lung function is selected from the group consisting of decreased peak airflow, decreased vital capacity, weakened respiratory muscles, reduced elasticity of bronchial tubes and air sacs, desensitized coughing nerves, and increased inflammation. In another preferred embodiment, the improvement in lung function in brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. In another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In the most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject. In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal.

In another most preferred embodiment, the invention discloses a method for delaying lung senescence in an ageing subject comprising a) identifying an aged subject showing signs of lung ageing, b) administering an effective dose of a composition comprising Rosmarinic acid, to bring about reduction in signs of lung ageing. In a related embodiment, the composition further comprises *Nigella sativa* extract standardised to contain not less than 5% w/w thymoquinone . In a related embodiment, the signs of lung ageing is selected from the group consisting of decreased peak airflow, decreased vital capacity, weakened respiratory muscles, reduced elasticity of bronchial tubes and air sacs, desensitized coughing nerves, and increased inflammation. In another preferred embodiment, delay in lung senescence is brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. In another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject. In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal.

In another most preferred embodiment, the invention discloses a composition comprising Rosmarinic acid for use in improving lung function in a subject showing signs of decreased lung function. In a related embodiment, the composition further comprises *Nigella sativa* extract standardised to contain not less than 5% w/w thymoquinone. In a related embodiment, lung function is decreased in conditions selected from the group consisting of, but not limited to, restrictive, obstructive or mixed lung disease or ageing. In another related embodiment, restrictive lung diseases include Idiopathic pulmonary fibrosis (IPF), Non-specific interstitial pneumonia (NSIP), Cryptogenic organizing pneumonia (COP), Sarcoidosis, Acute interstitial pneumonia (AIP), decrease in lung function due to inorganic dust exposure such as silicosis, asbestosis, talc, pneumoconiosis, berylliosis, hard metal fibrosis, coal worker's pneumoconiosis, chemical worker's lung, Organic dust exposure such as farmer's lung, bird fancier's lung, bagassosis, and mushroom worker's lung, humidifier lung, hot tub pneumonitis, Hypersensitivity pneumonitis, Systemic sclerosis, Pulmonary vasculitis, Pulmonary Langerhans cell histiocytosis, conditions due to medications such as nitrofurantoin, amiodarone, gold, phenytoin, thiazides, hydralazine, bleomycin, carmustine, cyclophosphamide, methotrexate and conditions due to radiation therapy. In another related embodiment, obstructive lung disease includes Chronic obstructive pulmonary disease (COPD), which includes emphysema and chronic bronchitis, Asthma, Bronchiectasis and Cystic fibrosis. In a related embodiment, the signs of decreased lung function is selected from the group consisting of decreased peak airflow, decreased vital capacity, weakened respiratory muscles, reduced elasticity of bronchial tubes and air sacs, desensitized coughing nerves, and increased inflammation. In a related embodiment, the signs of decreased lung function is selected from the group consisting of decreased peak airflow, decreased vital capacity, weakened respiratory muscles, reduced elasticity of bronchial tubes and air sacs, desensitized coughing nerves, and increased inflammation. In another preferred embodiment, the improvement in lung function in brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. IN another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In the most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject . In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal. In another most preferred embodiment, the invention discloses a composition comprising Rosmarinic acid for use in delaying lung senescence in an ageing subject. In a related embodiment, the composition further comprises *Nigella sativa* extract standardised to contain not less than 5% w/w thymoquinone . In a related embodiment, the signs of lung ageing is selected from the group consisting of decreased peak airflow, decreased vital capacity, weakened respiratory muscles, reduced elasticity of bronchial tubes and air sacs, desensitized coughing nerves, and increased inflammation. In another preferred embodiment, delay in lung senescence is brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. In another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In the most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject. In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal.

In another most preferred embodiment, the invention discloses use of a composition comprising Rosmarinic acid in improving lung function in a subject showing signs of decreased lung function. In a related embodiment, the composition further comprises *Nigella sativa* extract standardised to contain not less than 5% w/w thymoquinone. In a related embodiment, lung function is decreased in conditions selected from the group consisting of, but not limited to, restrictive, obstructive or mixed lung disease or ageing. In another related embodiment, restrictive lung diseases include Idiopathic pulmonary fibrosis (IPF), Non-specific interstitial pneumonia (NSIP), Cryptogenic organizing pneumonia (COP), Sarcoidosis, Acute interstitial pneumonia (AIP), decrease in lung function due to inorganic dust exposure such as silicosis, asbestosis, talc, pneumoconiosis, berylliosis, hard metal fibrosis, coal worker's pneumoconiosis, chemical worker's lung, Organic dust exposure such as farmer's lung, bird fancier's lung, bagassosis, and mushroom worker's lung, humidifier lung, hot tub pneumonitis, Hypersensitivity pneumonitis, Systemic sclerosis, Pulmonary vasculitis, Pulmonary Langerhans cell histiocytosis, conditions due to medications such as nitrofurantoin, amiodarone, gold, phenytoin, thiazides, hydralazine, bleomycin, carmustine, cyclophosphamide, methotrexate and conditions due to radiation therapy. In another related embodiment, obstructive lung disease include Chronic obstructive pulmonary disease (COPD), which includes emphysema and chronic bronchitis, Asthma, Bronchiectasis and Cystic fibrosis. In a related embodiment, the signs of decreased lung function is selected from the group consisting of decreased peak airflow, decreased vital capacity, weakened respiratory muscles, reduced elasticity of bronchial tubes and air sacs, desensitized coughing nerves, and increased inflammation. In a related embodiment, the signs decreased lung function is selected from the group consisting of decreased peak airflow, decreased vital capacity, weakened respiratory muscles, reduced elasticity of bronchial tubes and air sacs, desensitized coughing nerves, and increased inflammation. In another preferred embodiment, the improvement in lung function in brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. In another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In the most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject. In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal.

In another most preferred embodiment, the invention discloses use of a composition comprising Rosmarinic acid in delaying lung senescence in an ageing subject. In a related embodiment, the composition further comprises *Nigella sativa* extract standardised to contain not less than 5% w/w thymoquinone. In a related embodiment, the signs of lung ageing is selected from the group consisting of decreased peak airflow, decreased vital capacity, weakened respiratory muscles, reduced elasticity of bronchial tubes and air sacs, desensitized coughing nerves, and increased inflammation. In another preferred embodiment, delay in lung senescence is brought about by improvement in respiratory muscle function, reduction of senile emphysema, regulation of cellular senescence markers, and defence against pathogens by increasing immunity. In another related embodiment, improvement in respiratory muscle function is brought about by increasing tidal volume, minute ventilation, Transdiaphragmatic pressure (Pdimax) and maintaining normal respiratory rate. In another related embodiment, regulatory of cellular senescence is brought about by increasing Sirt-1 (Silencing information regulator 2 related enzyme 1) and AMPK Activation and decreasing mTOR (mammalian target of rapamycin) activity. In yet another related embodiment, reduction of senile emphysema is brought about by increasing the expression of laminin. In another related aspect, increase in host defense against pathogens is brought about by increasing immunoglobulin A (IgA). In yet another related embodiment, the effective dose of Rosmarinic acid is in the range of 10 - 40 mg/kg bodyweight of the subject or preferably the effective dose of Rosmarinic acid is in the range of 20 - 30 mg/kg bodyweight of the subject. In the most preferred embodiment, the effective dose of Rosmarinic acid is preferably 25 mg/kg bodyweight of the subject. In yet another related embodiment, the effective dose of *Nigella sativa* extract is in the range of 10 - 40 mg/kg bodyweight of the subject or preferably the effective dose of *Nigella sativa* extract is in the range of 20 - 30 mg/kg bodyweight of the subject. In the most preferred embodiment, the effective dose of *Nigella sativa* extract is preferably 25 mg/kg bodyweight of the subject. In yet another related embodiment, the effective dose of a composition comprising Rosmarinic acid and *Nigella sativa* extract is in the range of 20 - 80 mg/kg bodyweight of the subject or preferably the effective dose of a composition comprising Rosmarinic acid and *Nigella sativa* extract is in the range of 30 - 70 mg/kg bodyweight of the subject or preferably the effective dose of a composition comprising Rosmarinic acid and *Nigella sativa* extract is in the range of 40 - 60 mg/kg bodyweight of the subject. In the most preferred embodiment, the effective dose of a composition comprising Rosmarinic acid and *Nigella sativa* extract is preferably 50 mg/kg bodyweight of the subject. In a related embodiment of the invention, the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables. In another embodiment, the subject is a mammal.

The preferred embodiments are further described by means of the following examples. Though the present composition is effective in improving lung function across different groups, the examples hereto illustrate the improvement in lung function either physically or biologically ageing subjects. The examples are provided solely for illustration and are not to be construed as limitations of the present disclosure, as many variations thereof are possible without departing from the spirit and scope of the present disclosure.

### Example 1: Isolation of Rosmarinic acid

Rosmarinic acid used in the present invention can be isolated from different plant sources, specifically from the plant parts of Lamiaceae family. Initially, stabilization of the leaves is essential for increasing the yield of rosmarinic acid. The stabilization was done using the below process:

A process of preparing plant part of Lamiaceae family, preserving and stabilizing RA for isolation Lamiaceae family comprising steps of:
a) Charging 1000 kgs of plant part from Lamiaceae into Masher machine in lots;
b) Preparing acid solution 2.5 M (15 L of Hydrochloric acid in 60 L of Water) and slowly charge the solution to Masher machine along with 1000 kgs of leaves to make uniform mixture;
c) Collecting the leaves paste and leave the paste for 5 hrs;
d) Charging the paste from step c to Masher machine;
e) Preparing base 0.12M (250 g of Sodium bicarbonate in 25 L of Water) and slowly charge the solution to Masher machine along with Coleus leaves paste from step d to make uniform mixture;
f) Collecting the paste from step e and spread them on open yard for sun drying;
g) Collecting the dried material (~100 kg coarse powder) from step f for isolation of RA.

Further, Rosmarinic acid was isolated using the steps:
a. Charging 3 volumes of Ethanol (300 L) to the reactor containing coarse powder of leaves and stirred at 60-65°C for three hours;
b. Filtering the mass from step a through Nutsche filter;
c. Repeating steps a and b for two more times;
d. Collecting all the filtrate and concentrate into a thick paste and strip off all the ethanol;
e. Dissolving the concentrated extract from step d with 12 volumes of demineralized water (DM) (300-360 L) and stir for 3 hrs at 60°C, then cool it to room temperature;
f. Adjusting pH to 2.8 - 3.2 with 10% Sodium bicarbonate solution/50% Hydrochloric acid (HCl) stir for 15 mins, reconfirm the pH and filter through hyflo bed;
g. Extract the aqueous layer with one third volume of Ethyl acetate (3 times);
h. Concentrate the Ethyl acetate portion completely and note down the quantity;
i. Dissolve the material obtained from Step h with 10 volumes of DM water and adjust pH of the aqueous portion to 6.8 to 7.2 with 10% Sodium bicarbonate solution and filter the mass through hyflo bed;
j. Partition the aqueous portion with one-third volume of ethyl acetate or chloroform ( × 3 times) and take the organic layer for solvent recovery;
k. Adjust pH to 2.8 to 3.2 with 50% HCl of the aqueous portion obtained from step j;
l. Pass the aqueous portion through Hyflo bed;
m. Partition the aqueous portion obtained from step l with one-third volume of ethyl acetate (× 3 times);
n. Discard the aqueous layer after confirming the yield, concentrate ethyl acetate to pourable mass, and dry in Vacuum tray dryer (VTD) at 60-65 ° C to get powder;
o. Extract the above material with 12 volumes of 20% salt-water solution, and repeated 3 times at 80-85 °C, each extraction 2 hrs.;
p. Combine all the aqueous extract and pass through Hyflo bed;
q. Extracting the clear filtrate with one-third volume of Ethyl acetate (× 3 times);
r. Combine all Ethyl acetate portion obtained from step q and wash with cold water;
s. Distill off the solvent completely and dry at 60-65°C to get powder;
t. Charge 12 volumes of DM water, stir at RT until complete solubilization of the extract obtained;
u. Reduce the volume to six and store at 4-5°C for 48 hrs;
v. Filter the crystals and wash with cold water. Suck dry the product for 5-6hrs at RT and final drying at 65°C in VTD;
w. Isolate rosmarinic with an yield of at least 0.5 kg as a Creamish white crystalline powder at 90% w/w NLT.

The effect on acid initial acid / base treatment was evaluated by determining the yield of Rosmarinic acid in Coleus and Plectranthus leaves using the above process. The yield of rosmarinic acid without stabilizing the leaves (without acid treatment) was 15% w/w (assay of 0.06%w/w) in Coleus leaves and 13% w/w (assay of 0.11 % w/w) in Plectranthus leaves. Initial acid treatment increased the yield of Rosmarinic acid to 31% w/w (assay 3.72% w/w) in Coleus leaves and 32% w/w (assay 8.17% w/w) in Plectranthus leaves. This shows that the initial acid treatment of leave was very useful in stabilizing the Rosmarinic acid content, thereby increasing the yield of Rosmarinic acid.

### Example 2: Effect of Rosmarinic acid and Nigella sativa on improving lung function

### Materials:

The Rosmarinic acid (RA) and *Nigella sativa* combination is trademarked as Rosmagellin^{™}. Rosmarinic acid used in the present invention can be isolated from different plant sources, specifically from the plant parts of Lamiaceae family, using methods and processes known in state of art or is chemically synthesised. The composition comprising not less than 5% w/w thymoquinone is isolated from *Nigella sativa* (Nigellin^{®}) using similar process steps outlined in patent no. US10945969.

### Example 2a: Experimental design

The experiments were performed in old C57bl/6 mice (28-30 weeks). Table 1 discloses the groups of mice used for the experimentation. C57BL/6 mice were bred and naturally aged in a pathogen-free colony. Mice were group-housed six per cage for the treatment regimen and maintained on a 12-hr light-dark schedule with ad libitum access to food and water. All experiments were designed according to the principles of animal use for gerontological research (Miller, R. A., & Nadon, A. L. (2000). Principles of animal use for gerontological research. Journals of Gerontology. Series A, Biological Sciences and Medical Sciences, 55, B117-B123)

**Table 1: Experimental groups**

| **Group** | **Treatment groups** |
|---|---|
| I | Old Mice (control) |
| II | Rapamycin (8 mg/kg p.o.) |
| III | Rosmarinic acid (25 mg/kg p.o.) |
| IV | *Nigella sativa* extract (25 mg/kg p.o.) |
| V | Rosmarinic acid (25 mg/kg p.o.) + *Nigella sativa* extract (25 mg/kg p.o.) |

The above test substances were administered to the mice for a period of 90 days. Rapamycin was used as a positive control. After 90 days of administration experiments were performed to assess the respiratory muscle function, anatomical changes, cellular senescence and immunological changes.

### Example 2b: Assessment of respiratory muscle function:

The respiratory muscle function in the study animals was assessed by estimating the Tidal Volume, Minute ventilation, Transdiaphragmatic pressure (Pdimax) and Respiratory rate.

**Tidal Volume** - Tidal volume is the amount of air that moves in or out of the lungs with each respiratory cycle. It is an important determinant in many different breathing functions (Hallet et al., Physiology, Tidal Volume, StatPearls Publishing*).* Low tidal volume can cause a number of symptoms which include Difficulty breathing (dyspnea) at rest, Excessive daytime/nighttime sleepiness, Anxiety, Delirium, and Lethargy (Kristin Hayes, What Is Tidal Volume? Lung Health, Verywellhealth, August 2023, https://www.verywellhealth.com/tidal-volume-509025, accessed 10 August 2024*).* Lower tidal volumes are prevalent in ageing subject or subject with compromised lung function leading to breathing difficulties. In the present study, ventilatory parameters were measured in awake mice during eupnoea (spontaneous breathing of room air) using a whole-body plethysmography system . Before all recordings, the system was calibrated according to the manufacturer's recommendations. Recordings were taken after 10 min of acclimatization and recorded for one hour. Recorded parameters included tidal volume and minute ventilation

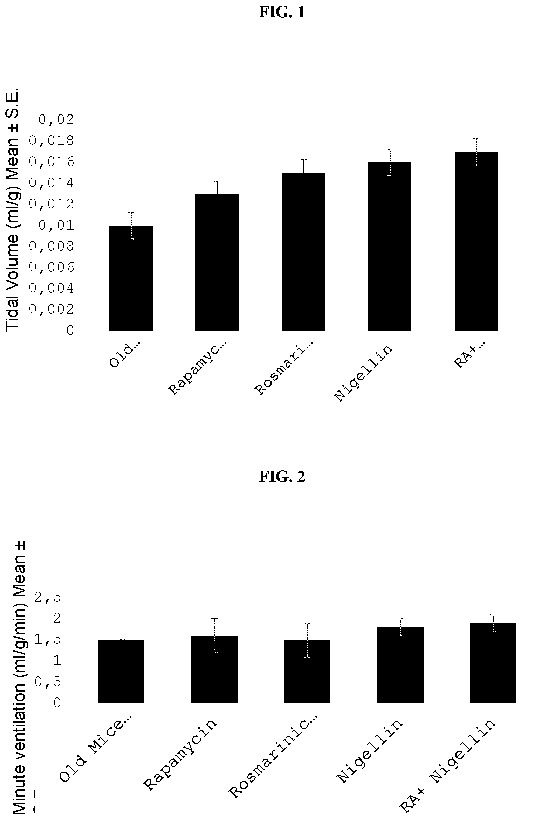

The results indicated that the tidal volume of older mice was lower and increased with the administration of Rosmarinic acid and *Nigella sativa* extract, either individually and in combination (Fig. 1). It is clearly evident that individual treatment of Rosmarinic acid (Rosinin^{™}) or *Nigella sativa* extract (Nigellin^{®}) is better than control, and Rapamycin. Further the combination is better than individual treatment.

**Minute ventilation** - Minute ventilation is the amount of air inhaled or exhaled from a person's lungs in one minuteis an important measurement that's related to tidal volume. In a normal healthy subjects, minute volume while resting is about 6-8 litres per minute and for a typical adult it ranges around 4 to 6 litres in 60 seconds *(*Kristin Hayes, What Is Tidal Volume? Lung Health, Verywellhealth, August 2023*).* The older mice in the current investigation showed lower minute ventilation, showing signs of ageing. Administration of Rosmagellin^{™} increased the minute ventilation and improved the respiratory rate (Fig. 2). Furthermore, treatment of Rosmarinic acid (Rosinin^{™}) or *Nigella sativa* extract (Nigellin^{®}) exhibited better improvement than control, and Rapamycin. Further the combination is better than individual extracts.

**Transdiaphragmatic pressure (Pdimax)** - The diaphragm muscle is the most important contractile district used for breathing. It generally provides sufficient forces to accomplish a range of functions including ventilatory and high force non-ventilatory motor behaviours like sneezing, gagging and coughing (Greising et al., Functional Measurement of Respiratory Muscle Motor Behaviours using Transdiaphragmatic Pressure, Methods Mol Biol. 2016; 1460: 309-319). Like other muscles in the human body, it is subject to ageing. For the present study, C57BL/6 were anesthetized by an intraperitoneal injection of fentanyl (0.3 mg/kg) and diazepam (5 mg/kg). Anesthetized mice were monitored continually for temperature, oxygen saturation, and heart rate. Palpebral reflex and pain response were monitored to ensure depth of anaesthesia. Briefly, a tracheostomy was performed, and the trachea was cannulated (*Greising, Mantilla, et al., 2013).* Spontaneous ventilation was maintained, the abdomen was bound, and Pdi was recorded using solid-state pressure catheters. The catheters were inserted through the mouth and positioned in the oesophagus and stomach. The placement of catheters was confirmed by positive deflection of the stomach signal and negative deflection of the oesophageal signal across behaviours. The tension of the abdominal binding was adjusted until optimal gastric and esophageal pressures were achieved during eupnoea. For both esophageal and stomach signals during stimulation, it was verified that there were no movement artifacts, and the duration and stimulation period of the Pdi response were the same. Recordings of Pdi across behaviours were obtained during spontaneous breathing of room air (eupnoea) for 5 min. The pressure data were collected using a PowerLab 8/35 data-acquisition system and analyzed using LabChart (Greising, S. M., Sieck, D. C., Sieck, G. C., & Mantilla, C. B. (2013). Novel method for transdiaphragmatic pressure measurements in mice. Respiratory Physiology & Neurobiology, 188, 56 59)

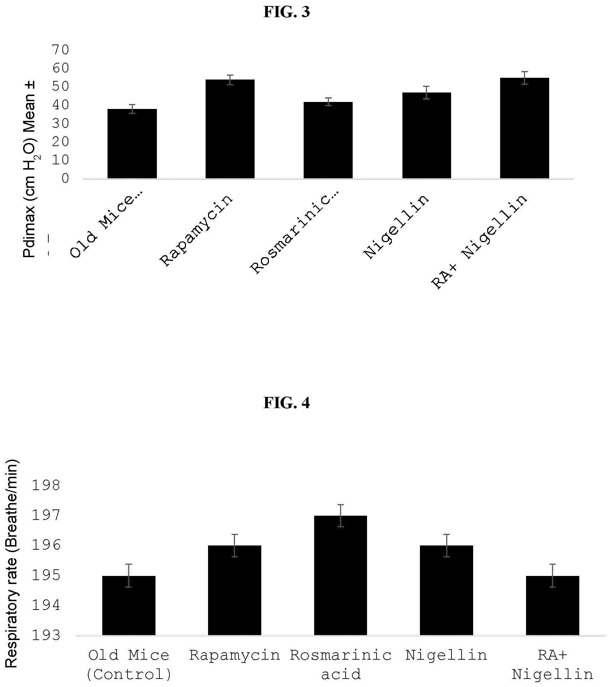

The results indicated that Pdimax was sufficiently lower in the aged mice suggesting reduced respiratory assistance, which was increased by the administration of Rosmagellin ^{™} (Fig. 3).

**Respiratory rate** - The respiratory rate is generally constant with adults reporting around 12-20 breaths per minute. It is reported to increase with age (Takayama et al., Aging is independently associated with an increasing normal respiratory rate among an older adult population in a clinical setting: A cross-sectional study, Geriatr Gerontol Int, 2019;19(11):1179-1183). However, in the current study, the respiratory rate was not significantly altered by the administration of Rosmarinic acid (Rosinin ^{™}) and Nigellin^{®} (Rosmagellin ^{™} ) (Fig. 4).

**Example 2b: Assessment of anatomical changes and senile emphysema** - Hyperinflation of the lungs may occur with ageing along with destruction of lung parenchyma or loss of supporting structures within the lung parenchyma (Gillooly M, Lamb D. Airspace size in lungs of lifelong non-smokers: effect of age and sex. Thorax. 1993;48:39-43). Generally, after 50 years degeneration of the elastic fibers occurs homogenously around the alveolar duct resulting in enlargement of airspaces. Reduction in these elastic fibers results in premature closure of small airways during normal breathing and can potentially cause air trapping and hyperinflation, hence "senile emphysema" (Sharma et al., Effect of aging on respiratory system physiology and immunology, Clin Interv. Aging. 2006 Sep; 1(3): 253-260*).* Additionally, it has been observed that the elastic recoil of the lung reduces with increasing age. This recoil is mainly supported by Laminin. Laminins are essential components of basement membranes, playing important roles in cell adhesion, proliferation, and differentiation. In the present study, the expression of laminins was made using the following protocol. After anesthetization by chloral hydrate, blood samples from the test animals were collected by a small puncture to retro orbital plexus and centrifuged at 4000 × g for 10 min at 4 °C to separate the plasma for subsequent experiments. Thereafter, the lungs were immediately harvested and weighed and tissue supernatant was collected for analysis. Laminin was measured using commercially available ELISA Kit (AbCam ELISA Kit) according to the user's manual provided. The solid-phase sandwich ELISA (enzyme-linked immunosorbent assay) is designed to measure the amount of the target bound between a matched antibody pair. A target-specific antibody has been pre-coated in the wells of the supplied microplate. Samples, standards, or controls are then added into these wells and bind to the immobilized (capture) antibody. The sandwich is formed by the addition of the second (detector) antibody, a substrate solution is added that reacts with the enzyme-antibody-target complex to produce measurable signal. The intensity of this signal is directly proportional to the concentration of target present in the original specimen.

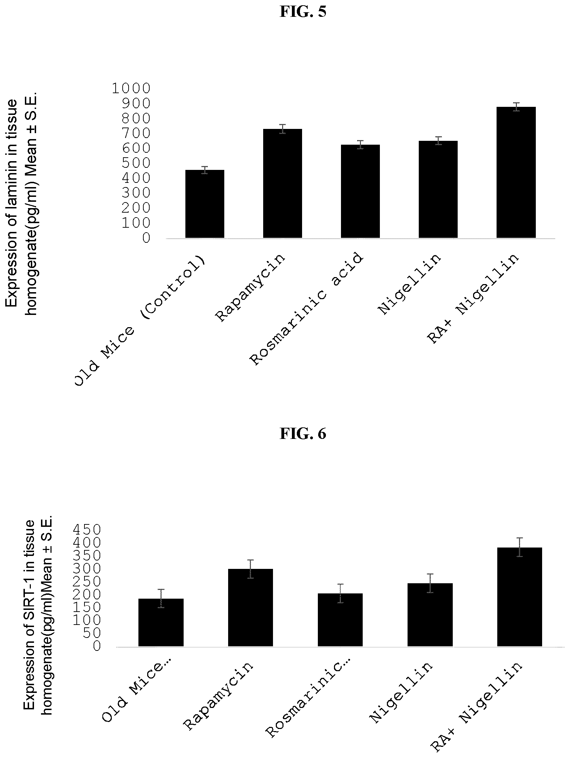

The study revealed that the expression was laminin was reduced in older mice and was increased by the administration of Rosmagellin^{™} individually and in combination (Fig. 5). This indicates that Rosmagellin ^{™} decrease senile emphysema and reverse the effect of ageing on the lungs.

### Example 2c: Assessment of cellular senescence

A number of cellular mechanisms and alterations constitute the hallmarks of cellular senescence. These include a stable cell cycle arrest chromatin alteration & reorganization; Inhibition of Sirt-1. Inhibition of AMPK and Dysregulation of mTOR. Silencing information regulator 2 related enzyme 1(sirtuin1, SIRT1) is an NAD+-dependent deacetylase, which is involved in the regulation of cellular senescence and aging. The expression of SIRT1 is diminished with aging in mice. By contrast, increased expression of SIRT1 is sufficient to extend lifespan in mice, yeast and *C.elegans.* Activation of SIRT1 can suppress the mechanisms of lung aging by inducing deacetylation of different substrates, thereby improving the progress of age-related lung diseases (Chen C, Zhou M, Ge Y, Wang X. SIRT1 and aging related signaling pathways, Mech Ageing Dev, 2020;187:111215*).* In the present study, SIRT 1 was estimated in lung tissue homogenate using commercially available ELISA kits (Abcam Elisa kits). The expression of SIRT1 was significantly compromised in aged mice which was improved by the administration of Rosmagellin ^{™} either individually or in combination (Fig. 6), this clearly suggesting anti-senescent activities of the composition

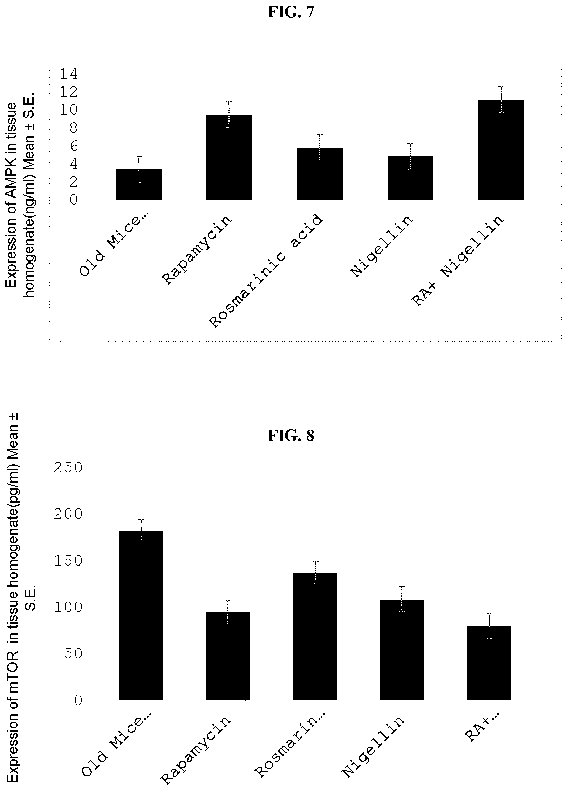

Activation of AMPK delays senescence. Abnormal autophagy and aging promote each other, and AMPK is an important regulator of this process. AMPK is involved in the transduction of several aging-related signaling pathways. AMPK intervenes in the development of many age-related diseases. The molecular targets, AMPK, mTOR were measured in lung tissue homogenate by commercially available ELISA kits according to the instructions provided in user's manual (Abcam Elisa kits) The AMPK levels were significantly lower in aged mice was significantly lower in aged mice which was increased by the administration of Rosmagellin ^{™} either individually or in combination (Fig. 7).

mTOR is well-known to inhibit autophagy, promoting the accumulation of protein aggregates and degenerated mitochondria leading to a deep dysregulation of mitochondrial functions. These events contribute to the pathogenesis of age-related disorders. The expression of mTOR was estimated using commercially available ELISA kits according to the instructions provided in user's manual (Abcam Elisa kits) In the lung tissue homogenate, the expression was mTOR was found to be significantly higher in aged mice, which was decreased by the administration of Rosinin^{™} and Nigellin^{®} individually or in combination (Fig.8).

### Example 2d: Assessment of immunological changes

Bronchoalveolar lavage (BAL) fluids in healthy older subjects have consistently shown an increased proportion of neutrophils and lower percentage of macrophages compared with younger adults. There is age-associated decline in immunoglobins IgA and IgM. Ratio of lymphocyte increases with age in the BAL fluid, suggesting the presence of primed T-cell from repeated antigenic stimuli of the lower respiratory tract mucosa. Immunoglobulin A (IgA) is the most abundant Ig in mucosae where it plays key roles in host defense against pathogens and in mucosal immunoregulation. The method of choice to analyze the number and composition of immune cells, and to detect secreted cytokines and other mediators in the lungs of experimental animals, is bronchoalveolar lavage (BAL). Tracheotomy, a partial cut of the trachea with a scalpel, or a hole punched into the tracheal wall with an injection needle was used as a method. This opening was used to insert a suitable cannula into the trachea. To prevent the cannula from slipping out of the trachea during BAL, and to minimize leakage of BAL fluid, the cannula was secured by tying medical yarn around the trachea and fixing it with a surgical knot. Using a 5-mL syringe 1 mL of PBS with protease and phosphatase inhibitor was slowly injected. Collection of BALF was done by slowly drawing the injected PBS back into the syringe. The collected BALF was then placed in a 2-mL microfuge tube on ice until BALF samples from all mice were collected. It was centrifuged at 2000 × g for 5 min at 4 °C (Kalidhindi RSR, Ambhore NS, Sathish V. Cellular and Biochemical Analysis of Bronchoalveolar Lavage Fluid from Murine Lungs. Methods Mol Biol. 2021;2223:201-215). The estimation of IgA was done by commercially available ELISA kits according to the instructions provided in user's manual (ThermoFisher Elisa kits)

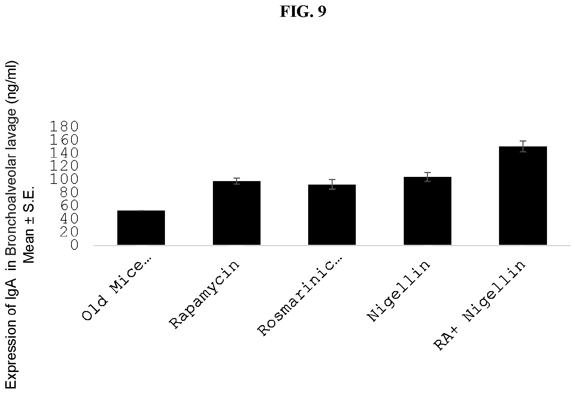

The levels were found to be significantly lower in aged mice, indicating lower immunity and host defense against pathogens. Administration of Rosmarinic acid and Nigellin^{®} either individually or in combination increased the expression of IgA thereby conferring immunity and increases defense against harmful pathogens (Fig. 9).

Overall, The formulation of Rosinin^{™} (25mg/kg p.o.) and Nigellin^{®} (25mg/kg p.o.) either individually or as a combination significantly improved the following features in the aged lungs of the rodents to show anti-ageing properties:
a. Improvement in respiratory muscle function (Fig. 1 to Fig. 4)
b. Reduction of senile emphysema (Fig. 5)
c. Regulation of Cellular senescence markers (Fig. 6 to Fig. 8)
d. Increase in host defense against pathogens (Fig. 9)

The composition can be effectively administered to prevent lung ageing and to increase lung function in older adults. The approximate human dosage will be in range of 200-1000 mg/day, preferably at 250 mg/day. It is to be noted that any dose range that brings about the said effect of improving lung function and decreasing sign of lung ageing shall be considered equivalent. It will be obvious to use this composition for different subjects, not limited to older adults.

### Example 3: Formulations

Tables 2-10 provide illustrative examples of nutraceutical formulations

**Table 2: Tablet**

| **Active Ingredients** | |
|---|---|
| | Rosmarinic Acid (Rosinin^{™}) |

| **Excipients** | |
|---|---|
| | Microcrystalline cellulose, Colloidal silicon dioxide, Magnesium stearate, BioPerine^{®}, Polyvinylpyrrolidone / starch / Hydroxy propyl methyl cellulose, Hydroxy propyl cellulose |

**Table 3: Tablet**

| **Active Ingredients** |
|---|
| Rosmarinic acid (Rosinin^{™}) |
| Nigella sativa extract (Nigellin^{®}) |

| **Excipients** |
|---|
| Microcrystalline cellulose |

**Table 4: Capsule**

| **Active Ingredients** |
|---|
| Rosmarinic acid (Rosinin^{™}) |

| **Excipients** |
|---|
| Microcrystalline cellulose, Colloidal silicon dioxide, Magnesium stearate, BioPerine^{®}, Polyvinylpyrrolidone / starch / Hydroxy propyl methyl cellulose, Hydroxy propyl cellulose |

**Table 5: Capsule**

| **Active Ingredients** |
|---|
| Rosmarinic acid (Rosinin^{™}) |
| Nigella sativa extract (Nigellin^{®}) |

| **Excipients** |
|---|
| Microcrystalline cellulose, Colloidal silicon dioxide, Magnesium stearate, Polyvinylpyrrolidone / starch / Hydroxy propyl methyl cellulose, Hydroxy propyl cellulose |

**Table 6: Powder**

| **Active Ingredients** |
|---|
| Rosmarinic acid (Rosinin^{™}) |
| Nigella sativa extract (Nigellin^{®}) |

| **Excipients** |
|---|
| BioPerine^{®}, |

**Table 7: Powder**

| **Active Ingredients** |
|---|
| Rosmarinic acid (Rosinin^{™}) |
| Nigella sativa extract (Nigellin^{®}) |

| **Excipients** |
|---|
| BioPerine^{®}, |

**Table 8: Gummy Formulation**

| **Active Ingredients** |
|---|
| Rosmarinic acid (Rosinin^{™}) |

| **Excipients** |
|---|
| BioPerine^{®}, Gelatin (270 Bloom Mesh 10), Refined Sugar, Glucose Corn Syrup, Citric Acid, Lactic Acid, Water, Natural Mango Flavor M38630, Tartaric Acid, Refined Sugar |

**Table 9: Gummy Formulation**

| **Active Ingredients** |
|---|
| Rosmarinic acid (Rosinin^{™}) |
| Nigella sativa extract (Nigellin^{®}) |

| **Excipients** |
|---|
| Gelatin (270 Bloom Mesh 10), Refined Sugar, Glucose Corn Syrup, Citric Acid, Lactic Acid, Water, Natural Mango Flavor M38630, Tartaric Acid, Refined Sugar |

**Table 10: Candy Formulation**

| **Active Ingredients** |
|---|
| Rosmarinic acid (Rosinin^{™}) |

| **Excipients** |
|---|
| BioPerine^{®}, Sucrose, Liquid Glucose, Flavoring agent, Menthol, Acidulants (Citric acid / Tartaric Acid / Maleic Acid), Purified water |

**Table 11: Candy Formulation**

| **Active Ingredients** |
|---|
| Rosmarinic acid (Rosinin^{™}) |
| Nigella sativa extract (Nigellin^{®}) |

| **Excipients** |
|---|
| Sucrose, Liquid Glucose, Flavoring agent, Menthol, Acidulants (Citric acid / Tartaric Acid / Maleic Acid), Purified water |

The above formulations are just illustrative examples, any formulation containing the above active ingredient intended for the said purpose will be considered equivalent.

Other modifications and variations of the invention will be apparent to those skilled in the art from the foregoing disclosure and teachings. Thus, while only certain embodiments of the invention have been specifically described herein, it will be apparent that numerous modifications may be made thereto without departing from the spirit and scope of the invention.

## Claims

1. A composition comprising Rosmarinic acid for use in improving lung function in a subject showing signs of decreased lung function.

2. A composition comprising Rosmarinic acid for use in delaying lung senescence in an ageing subject.

3. The composition as in claim 1 or claim 2, wherein the composition further comprises *Nigella saliva* extract standardised to contain not less than 5% w/w thymoquinone.

4. The composition as in claim 1 or claim 2, wherein the signs of decreased lung function and lung ageing are selected from the group consisting of decreased peak airflow, decreased vital capacity, weakened respiratory muscles, reduced elasticity of bronchial tubes,air sacs, desensitized coughing nerves, and increased inflammation.

5. The composition as in claim 1 or claim 2, wherein the improvement in lung function and delay in lung senescence in brought about by improvement in respiratory muscle function, reduction of senile emphysema, regulation of cellular senescence markers, and defence against pathogens by increasing the immunity.

6. The composition as in claim 5, wherein the improvement in respiratory muscle function is brought about by increasing tidal volume, minute ventilation, Transdiaphragmatic pressure (Pdimax) and maintaining normal respiratory rate.

7. The composition as in claim 5, wherein the regulation of cellular senescence is brought about by increasing Sirt-1 (Silencing information regulator 2 related enzyme 1), AMPK Activation and decreasing mTOR (mammalian target of rapamycin) activity.

8. The composition as in claim 5, wherein the reduction of senile emphysema is brought about by increasing the expression of laminin.

9. The composition as in claim 5, wherein increase in defense against pathogens is brought about by increasing immunoglobulin A (IgA) levels.

10. The composition as in claim 1 or claim 2, wherein the composition is formulated with a pharmaceutically/nutraceutically acceptable excipients, adjuvants, diluents or carriers, and administered orally in the form of tablets, capsules, syrups, gummies, powders, suspensions, oil, emulsions, chewables, candies, and eatables.

11. The composition as in claim 1 or claim 2, wherein the subject is a mammal.
